# EUROPEAN PATENT SPECIFICATION

(11) **EP 4 303 323 B1**
(45) Date of publication and mention of the grant of the patent: **19.02.2025**
(21) Application number: 22460030.4
(22) Date of filing: 05.07.2022
(51) Int. Cl.: C12Q 1/6886

(54) **A METHOD DIFFERENTIATING BENIGN AND MALIGNANT TYROID NODULES**
VERFAHREN ZUM UNTERSCHEIDEN VON GUTARTIGEN UND BÖSARTIGEN TYROID-KNÖTCHEN
PROCÉDÉ DE DIFFÉRENCIATION DE DIFFÉRENCIATION DE NODULES THYROÏDIENS BÉNINS ET MALINS

(43) Date of publication of application: 10.01.2024
(73) Proprietor: Narodowy Instytut Onkologii im. Marii Sklodowskiej-Curie Panstwowy Instytut Oddzial w Gliwicach, 44-102 Gliwice (PL); Politechnika Slaska, 44-100 Gliwice (PL); Polskie Towarzystwo Endokrynologiczne, 02-097 Warszawa (PL); Uniwersytet Medyczny w Lodzi, 90-419 Lodz (PL); WASKO Spólka Akcyjna (WASKO S.A.), 44-100 Gliwice (PL)
(72) Inventor: Student, Sebastian, 44-100 Gliwice (PL); Tyszkiewicz, Tomasz, 44-100 Gliwice (PL); Jarzab, Michal, 41-807 Zabrze (PL); Slowinska-Klencka, Dorota, 90-419 Lódz (PL); Rusinek, Dagmara, 44-100 Gliwice (PL); Krajewska, Jolanta, 44-100 Gliwice (PL); Wilk, Agata, 44-100 Gliwice (PL); Lakomiec, Krzysztof, 44-100 Gliwice (PL); Zebracka, Jadwiga, 44-100 Gliwice (PL); Pluciennik, Alicja, 44-100 Gliwice (PL); Fujarewicz, Krzysztof, 43-100 Tychy (PL); Tarnawski, Rafal, 44-100 Gliwice (PL); Oczko-Wojciechowska, Malgorzata, 41-808 Zabrze (PL); Jarzab, Barbara, 44-101 Gliwice (PL)

(56) References cited:
- EP-A1- 2 366 800
- US-A1- 2015 038 376
- US-B2- 10 672 504
- ZHANG KE ET AL: "Identification and validation of potential target genes in papillary thyroid cancer", EUROPEAN JOURNAL OF PHARMACOLOGY, vol. 843, 22 November 2018 (2018-11-22), pages 217 - 225, XP085569568, ISSN: 0014-2999, DOI: 10.1016/J.EJPHAR.2018.11.026

## Description

The invention relates to a method of differentiating benign and malignant thyroid nodules and is intended for use in the area of preoperative diagnosis.

Another subject of the invention is the use of a hybridization probe set to detect in a thyroid nodule sample the expression level of the corresponding gene in order to differentiate benign and malignant thyroid tumor

The normal follicular thyroid cell is highly differentiated, showing specialized features, including the ability to respond to thyrotropic hormone (TSH), iodine transporters, the ability to bind iodine with thyroid peroxidase (TPO) and store it as bound to thyroglobulin (Tg), and maintain follicular structure. Abnormal proliferation of thyroid cells can result in the formation of nodules, which can be benign or malignant. Malignant thyroid tumors comprise papillary, follicular, and anaplastic carcinoma. Medullary carcinoma of the thyroid gland originates from perifollicular C cells. Papillary thyroid carcinoma (PTC) is the most common type of differentiated thyroid cancer that originates from follicular thyroid cells, and the diagnosis of follicular thyroid carcinoma (FTC) poses the most significant diagnostic challenges preoperatively. These carcinomas - papillary and follicular - are known as differentiated thyroid carcinomas (DTC).

Existing diagnostic methods do not provide complete safety in assessing that we are dealing with a benign thyroid nodule, which can be managed without surgery without harming the patient.

Routine diagnosis of thyroid nodules is based on imaging evaluation by ultrasonography and cytological evaluation of material (biological sample) obtained by fine-needle aspiration biopsy (FNAB). The material (biological sample) includes tissue fragments taken from the tumor by puncture and/or aspiration.

Currently, the cytological examination results are used to determine further clinical management. When the result suggests the suspicion of a malignant lesion, surgical treatment is required, while when a benign lesion is diagnosed, one usually remains on observation. The Bethesda system (TBSRTC - The Bethesda System for Reporting Thyroid Cytology) is used to determine the potential of the examined cells in the cytologic smear. It classifies the cytologic picture into six categories ranging from non-diagnostic lesions - B1 to malignant neoplasm - B6, where category B2 corresponds to a benign lesion, B3 - a follicular lesion of undetermined significance, category B4 - suspicoius for a follicular neoplasm, and B5 suspiciois for malignancy.

Fine-needle biopsy is the standard diagnostic procedure for suspicious thyroid nodules, but it has many limitations due to its inability to assess the arrangement of follicular cells within the thyroid follicle structure and to evaluate any invasive features. The amount of specimens obtained from the fine-needle biopsy is small, so immunohistochemical methods are difficult to apply. For this reason, nearly 30% of all biopsies of thyroid nodules are classified as indeterminate lesions, while only 15-30% of them are malignant lesions that require surgical intervention. It means that most of the resected nodules are benign and do not require radical medical management. Thus, the thyroid surgery performed may be considered ex-post unnecessary

Supportive testing for thyroid cytology, including widespread molecular diagnostics, could reduce the number of diagnostic surgeries, resulting in a better quality of life for the patient and less burden on the health care system. However, it is unclear to what extent gene-level analysis can help to state the diagnosis, as it may not capture the absolute differences between the genomes of malignant and benign forms of thyroid nodules, which are significantly affected by the classification parameters obtained. For example, in US2007037186A1, Jiang et al. revealed a 4-gene QRT-PCR test with a sensitivity of 92% for cancer diagnosis but a specificity of only 61%.

Studies on molecular alterations that play a role in the development of thyroid cancer have established that a point mutation V600E in the BRAF gene, leading to constitutive activation of this serine/threonine kinase, is the most common mutation in thyroid cancer, observed in about 45% of papillary thyroid carcinomas. RAS mutations, also involved in the development of thyroid tumors, are frequently found in follicular adenomas, suggesting their potential role in early follicular cell oncogenesis. Mutations of PTEN, PIK3CA, and other genes, as well as gene translocations (RET-PTC, PAX8- PPARG) and abnormal gene methylation, are considered critical genetic alterations. However, using some of these molecular markers in preoperative diagnosis is still insufficient to distinguish benign from malignant tumors with adequate sensitivity and specificity.

Gene classifiers based on assessing mutations of a few or even dozens of genes or evaluating the expression of a set of genes are well known. The most widespread is an expression classifier based on the study by Chudova et al., where the evaluation of the expression of a gene set using high-density oligonucleotide microarrays was used. The test is divided into several stages, and for a lesion deemed suspicious, an assessment of BRAF gene mutations and RET/PTC1 and RET/PTC3 rearrangements is additionally performed. It is a "rule out malignancy" test, and the result is primarily used to exclude malignancy of the nodule. Based on the results of this study, the Afirma Gene Expression Classifier test (Veracyte Inc, USA) was developed.

Recent advances in technology have influenced the introduction of a new version of the test using next-generation sequencing (NGS). The Genomic Sequencing Classifier (GSC) evaluates both gene expression and mutations. The test allows the analysis of several thousand genes, including, unlike GEC, detecting changes originating in Hürthle cells.

There are also known tests based solely on tumor mutation (DNA) analysis and similar tests with additional microRNA (miRNA) expression analysis to obtain high sensitivity and specificity.

There are no widely conducted validation tests involving the European population for the abovementioned gene classifiers.

The purpose of the present invention is to provide more effective differentiation between malignant and benign thyroid nodules.

The system for classifying a thyroid nodule as malignant or benign, known from the description of patent US9617604B2, is based on identifying sets of gene transcripts and involves determining the expression level of one or more transcripts in a sample, at least one of which contains the corresponding at least one option from SEQ ID Nos. 1-12, 261, 283-306, 657, 658 and 659. In addition, the classification system provides sets of target sequences and combinations of polynucleotide probes and derived primers that can be provided in a solution or an array.

The invention, known from EP2505664B1 patent description, relates to a method for distinguishing between malignant and benign thyroid nodules by performing RNA extraction in a standard FNAB specimen, particularly in an air-dried FNAB smear. The isolated RNA is analyzed for the presence of gene rearrangements and/or miRNA expression, with the presence of RET/PTC and/or PAX8/ PPARG rearrangements and/or mutations in genes encoding BRAF, N-, K-, and/or HRAS and/or differential miRNA expression in the sample classifying the tumor as malignant.

The US10236078B2 and US10672504B2 patent descriptions relate to methods for molecular profiling and diagnosis of genetic disorders and cancer, including gene expression markers associated with cancer or genetic disorders. Both inventions provide algorithms and methods for classifying thyroid cancer, determining molecular profiles, and analyzing the results to make a diagnosis.

US10672504B2 invention relates to compositions and methods for molecular profiling and diagnostics for genetic disorders and cancer, including but not limited to gene expression product markers associated with cancer or genetic disorders. In particular, the present invention provides algorithms and methods of classifying cancer, such as thyroid cancer, methods of determining molecular profiles, and methods of analyzing results to provide a diagnosis. A method for processing or analyzing a sample of thyroid tissue of a subject, comprising: (a) sequencing nucleic acid molecules from said sample of thyroid tissue to yield data comprising one or more levels of gene expression products in said sample of thyroid tissue, which one or more levels of gene expression products correspond to a plurality of genes comprising two or more genes selected from the group consisting of: ALK, CALCA, DICER1, IGF2BP3, MET, NTRK1, NTRK3, PAX8, PTEN, PTH and THADA; (b) using a trained algorithm in a computer to process said data from (a) to generate a classification of said sample of thyroid tissue as positive or negative for thyroid cancer at an accuracy of at least 85%, wherein said trained algorithm is trained with a plurality of training samples comprising known benign samples and known non-benign samples that is different from said sample of tissue; and (c) electronically outputting a report that identifies said classification of said sample of thyroid tissue as positive or negative for said thyroid cancer.

The use of genes showing differential expression in benign and malignant thyroid nodules for the diagnosis and staging of thyroid cancer is disclosed in the EP1756303B1 patent description. The EP3467123A2 invention concerns compositions, kits and methods for molecular profiling and diagnosis of cancer, including gene expression markers, alternative exon usage markers, and DNA polymorphisms associated with cancer, particularly thyroid cancer. Similarly, the description of patent US7901888B2 provides information on methods to diagnose, predict and stage thyroid cancer, using molecular markers. According to the disclosure of the invention US2015038376A1, as well as the international publication WO2013138726A1, biomarkers for thyroid nodule malignancy classification are selected from a group consisting of NPC2, S100A11, SDC4, CD53, MET, GCSH, and CHI3L1 using a quantitative real-time polymerase chain reaction (PCR) array. In addition, one or more reference genes are selected from the following group, including TBP, RPL13A, RPS13, HSP90AB1, and YWHAZ. The WO2009029266A2 patent relates to the use of differentially expressed thyroid genes (DETs) for diagnosing and staging thyroid cancer. Examples of DET genes include C21orf4 (DET1), Hs.145049 (DET2), Hs.296031 (DET3), KIT (DET4), LSM7 (DET5), SYNGR2 (DET6), C11orf8 (DET7), CDH1 (DET8), FAM13A1 (DET9), IMPACT (DET10), KIAA1128 (DET11).

From the description of the invention EP2366800A1 (national application PL390570A1), invention uses a gene set consisting of ANXA1, CHl3L1, CITED1, FN1, GDF15, LRP4, MET, PROS1, SFTPB, TPO, MPPED2, TFF3, DPP4 for diagnosing papillary thyroid cancer. In a sample of tumor tissue collected from a patient the gene expression profile is examined, wherein the expression profile for papillary thyroid cancer is the profile encompassing the increased expression of the following genes: ANXA1, CHI3L1, CITED1, FN1, GDF15, LRP4, MET, PROS1, SFTPB, MPPED2, TFF3 and DPP4 as well as the decreased expression of TPO.

A method for diagnosing a genetic disorder or cancer as known from the US20202974A patent includes detecting gene expression in a biological sample, comparing gene expression in the biological sample with a control sample, classifying the biological sample by inputting one or more differential gene expression levels into a trained algorithm; and identifying a biological sample as positive for a genetic disorder or cancer if the trained algorithm classifies the sample as positive for a genetic disorder or cancer at a certain level of specificity and/or sensitivity.

The invention, according to the EP2569626B1 patent, relates to components, kits, and methods of molecular profiling for diagnosing thyroid nodules. The method of evaluating a thyroid nodule sample involves determining the expression level of one or more genes, one or more of which correspond to at least five genes selected from SNCA, STK32A, THRSP, TIMP1, TIMP2, TMSB10, TNFRSF17, TNFRSF1A, TXNDC12, VWA5A, WAS, WIPI1 and ZFYVE16 and then classifying the thyroid nodule as benign or suspicious by comparing the gene expression levels of the sample with gene expression data for at least two different sets of biomarkers. Gene expression data for each set of biomarkers includes one or more reference gene expression levels correlated with the presence of one or more tissue types. The expression level is compared sequentially with gene expression data for the mentioned at least two different sets of biomarkers.

A method of diagnosing a thyroid nodule (benign/neoplastic), known from the PL406033A1 description of the invention, involves determining the amount of one or more microRNAs selected from a group including miR-146b-5p, miR-146b-3p, miR-221-5p, miR-221-3p, miR-222-5p, miR-222-3p, miR-181a-5p and miR-182-5p in a biological sample, and then comparing the expression of the microRNAs with the expression level of microRNAs in a control group.

According to the EP2925886B1 patent, a thyroid tissue sample is identified as malignant if the expression level of any one or more of the CXCR3, the CAR, and/or XB130 genes is decreased; and/or the expression level of any one or more of the CCR3, CXCL10, CK-19, TIMP-1, CLDN-1, and/or CCR7 genes is increased in the thyroid tissue sample as compared to the normal control expression level, wherein the total number of genes with increased or decreased expression is at least three.

Zhang KE et al. in a paper published in 2019 identified seven hub genes FN1, SERPINA1, ECM1, MMRN1, PROS1, CFD, and TIMP1 showing difference in expression between normal thyroid tissue and PTC that could be helpful for the development of gene panel for thyroid nodule diagnosis.

Unless otherwise specified, all technical and scientific terms used herein shall have the same meaning as commonly understood by a person of average skill in the field to which this invention belongs.

The terms used for the purposes of the present invention have the following meanings:
- Thyroid nodule - any focal lesion in the thyroid gland, benign or malignant, which can be subjected to the diagnostic process for definitive evaluation of the nature and clinical aggressiveness;
- Thyroid nodule sample (interchangeable with: tumor material, biological sample or specimen) - fresh and frozen samples, stored in liquid nitrogen or dry ice, and samples fixed with fixatives; taken by the FNAB method. The sample contains thyroid cells;
- Gene expression - the process by which the genetic information contained in a gene is read and transcribed into its products in the form of mRNA
- Evaluation of gene expression - intended means determining the amount or presence of mRNA transcript
- Gene expression profiling - the analysis of the expression of a set of genes
- Gene expression profile - it refers to both the entire set of information concerning a set of genes and information as to their expression, both considered as a whole and as a selected part, identifiable for a particular type of tissue or cell.
- Reference gene, control gene, normalizing gene - a gene whose expression is maintained at a constant level in normal and neoplastic cells regardless of their nature and phenotype
- PPV (positive predictive value) - the probability that an individual has a disease having a positive test result. Thus, if a positive test result is obtained, the PPV gives information on how sure an individual can be that he or she has the disease (based on the Clopper-Pearson method);
- NPV (negative predictive value) - the probability that an individual with a negative test result does not have a disease. So, if the person tested received a negative test result, NPV gives him information on how sure he can be that he does not have the disease;
- Sensitivity and specificity - values describing the ability (of a test) to detect the investigated feature (sensitivity) or detect its absence (specificity);
- ROC curve (Receiver Operating Characteristic Curve is a tool for evaluating the quality of a classifier, it provides a combined description of its sensitivity and specificity; TPR- True Positive Rate, the sensitivity of a classifier; FPR- False Positive Rate. False Positive Rate; a measure of a classifier's specificity, or specificity;
- Ct -The reaction cycle at which the fluorescence of a sample exceeds a cut-off line (threshold), set automatically or by the operator, is defined as Ct (cycle threshold). Ct is measured in a logarithmic increase in the amount of DNA, and the smaller its value is, the higher the concentration of array was at the beginning of the reaction.

The method according to the invention for differentiating benign and malignant thyroid nodules in tumor biopsy material includes the steps of RNA isolation, and determining the expression level of genes, and using a classifier, comparing the gene expression of a thyroid nodule sample with the gene expression of thyroid nodule samples in a training set.

It then identifies the thyroid nodule as malignant or benign and determines the probabilities of malignancy of the thyroid nodule, with the classifier trained on gene expression data correlated with the nature of the thyroid nodule, especially benign and malignant tumors including papillary and follicular thyroid carcinoma.

In addition, the expression level of a set of 32 genes is determined, which consist: AMOT, C3, CAMK2N1, CFI, DCSTAMP, DIO1, DUSP5, EGR1, EMP2, FAM20A, FAXC, FLRT3, HBG1, IGFBP6, IGSF1, ITGA2, KCNQ3, MET, METTL7B, MPZL2, NOD1, OLR1, PDE5A, PSD3, SDC4, SLC26A4, SLC26A7, SLC5A8, SLPI, TM4SF1, TPO, and TUSC3, and the normalization of the expression of each gene of this set is carried out under the endogenous control of the following six normalizing genes: EIF3A (EIF3S10), HADHA, UBE2D2, EIF5, PPIE, and RPLPO.

A nodule sample's malignant or benign character is determined with at least 91% specificity and at least 83% classification sensitivity for the normalized expression level of a set of 32 genes.

The nature of the nodule and the probability of malignancy of the tested sample are determined according to the decision rule of the classifier, taking into account the data on the expression level of the set of 32 genes and the gene expression level of each nodule sample in the training set, determined for each sample of this set for the same set of 32 genes and under the same normalization conditions.

The nature and probability of malignancy of a nodule sample are determined according to the criterion of maximum likelihood for a probabilistic model of normalized expression values of a set of 32 genes of the sample.

RT-qPCR, oligonucleotide microarray, or RNA-seq transcriptome sequencing methods are used to detect the expression levels of a set of genes.

Use of the set of hybridization probes Hs00611096_m1, Hs00163811_m1, Hs00218591_m1, Hs00989715_m1, Hs00229255_m1, Hs00174944_m1, Hs00244839_m1, Hs00152928_m1, Hs00171315_m1, Hs01034070_m1, Hs00383314_m1, Hs01922255_s1, Hs00361131_g1, Hs00181853_m1, Hs00169943_m1, Hs00158127_m1, Hs01120412_m1, Hs01565584_m1, Hs00378551_m1, Hs01083647_m1, Hs01036720_m1, Hs01552593_m1, Hs00153649_m1, Hs00939867_m1, Hs01120908_m1, Hs01070627_m1, Hs01104163_m1, Hs00377618_m1, Hs00268204_m1, Hs01547334_m1, Hs00892519_m1, Hs00185147_m1 for detecting in a thyroid nodule sample the expression level of a corresponding gene from a set of 32 genes AMOT, C3, CAMK2N1, CFI, DCSTAMP, DIO1, DUSP5, EGR1, EMP2, FAM20A, FAXC, FLRT3, HBG1, IGFBP6, IGSF1, ITGA2, KCNQ3, MET, METTL7B, MPZL2, NOD1, OLR1, PDE5A, PSD3, SDC4, SLC26A4, SLC26A7, SLC5A8, SLPI, TM4SF1, TPO, TUSC3 and for determining the nature of the nodule by the method according to claims. 1 to 3, for determining the nature of the nodule, using a computer software product having computer-readable support and compositions for measuring the normalized expression level of a set of genes, performing classification and determining the probability of the malignancy of the nodule sample.

For the purpose of carrying out the present invention, a set of genes that exhibit a specific pattern of altered expression in thyroid cancer is presented to support the differential diagnosis of thyroid nodules. The study uses fine-needle biopsy specimens of thyroid nodules of the Polish population with a full spectrum of diagnoses covering Bethesda categories from B2 to B6. The classification of a nodule sample determines the range of probability of malignancy, not just the dichotomous categorization (benign/malignant), which is important for further decisions. The gene set, considering the quality of the classification, is limited in number, which is essential for the cost of the test.

The method, according to the invention and related qualitative features of the classifier, as well as an example application in prediction, are shown in the following figures:
Fig. 1 shows a diagram that illustrates the selection of a probability cutoff threshold for an expression classifier by controlling its sensitivity and specificity, including the PPV and NPV parameters.

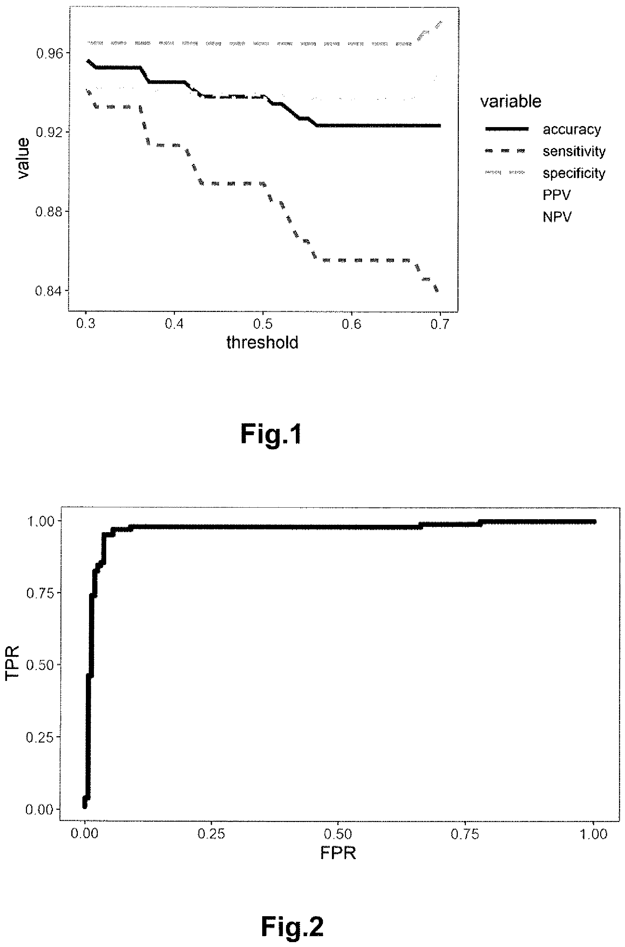

Fig 2. shows the quality characteristics of the classifier as a ROC curve for the samples tested. The ROC curve shows the characteristics of the sensitivity and specificity of the classifier. In the graph, TPR (Y-axis) expresses the sensitivity of the classifier, as the ratio of the number of observations of correctly classified positive cases (detection of thyroid cancer) to all observations correctly identified by the classifier, and is a number in the range of 0-1. In contrast, FPR (X-axis) expresses the ratio of all incorrectly classified positive cases (thyroid cancer labeled as a benign lesion) to the number of all observations labeled by the classifier as negative (benign lesions) and is calculated as specificity, and the number is in the range of 0-1. The shape of the ROC curve indicates that the classifier correctly labels benign/malignant.
Fig. 3 to Fig.7 illustrate in diagrams the use of the classifier to predict malignancy of a thyroid nodule.

Ultrasound examination is the first step in the diagnosis of a thyroid nodule after clinical evaluation. Depending on the estimated risk for a given image, the threshold size of the thyroid nodule for further evaluation is determined. A fine-needle aspiration biopsy of a thyroid nodule provides material for cytologic analysis, the result of which provides clinically useful data. The result categorized as Bethesda B3, B4, or B5 is subjected to additional study. B3, B4, and B5 nodules are collectively referred to as indeterminate nodules.

Clinical information based on the result of the cytologic examination provided by the pathologist is used to preselect thyroid nodules that qualify for additional molecular diagnostics using gene classifiers that assess only the change in gene expression. The clinical evaluation of the specimen, performed by a pathologist or team of pathologists, is a subjective evaluation based on the professional experience of the evaluating physician. In the case of improper clinical classification and erroneous preselection, there may be a discrepancy between the gene evaluation and clinical evaluation of the tumor sample.

Differentiating a thyroid nodule as benign or malignant by classifying it based on gene expression values requires an exploratory procedure and multivariate analysis with an initial fine-needle biopsy sample of the nodule containing the material for RNA isolation and an expression classifier.

More than 3,000 samples from fine-needle biopsies of thyroid nodules were collected for identifying genes for thyroid nodule differentiation using an independent test set, as well as for the earlier research phase and test set-up and execution. One or two additional samples were collected from each patient to obtain sufficient material to isolate nucleic acids for molecular analysis.

Total RNA was used in the study. Material collected by aspiration is protected in preservation buffer, frozen, and next RNA isolation and gene expression testing are performed using qPCR, microarray, or NGS techniques. Based on the transcript expression data, a molecular assay is done.

To implement the invention, the computer system provides an expression classifier as a product of computer software, having a computer-readable medium and the resources to carry out classification, modeling, and gene expression measurement methods. For this purpose, it has at least one processor and at least one program memory that contains the processor's execution instructions (classifier algorithm) for analyzing data and determining gene expression levels. Based on the classifier, decision rules are generated. In addition, it contains datasets for testing fine-needle biopsy samples of thyroid nodules. As a predictive model, the classifier can distinguish and characterize samples based on the expression of a designated set of genes.

The classifier, based on a Support Vector Machine (SVM) model with a radial kernel function and trained on measured gene expressions for a population of training samples, enables the prediction of sample class and determination of malignancy probability. The samples used for training are a part of the model and are called support vectors.

The classifier was validated using the bootstrap method (500 iterations). The class suggested by the classification describes two states: benign lesion or malignant lesion, whereby the test can be a cancer exclusion or confirmation test as needed.

To calculate the probability of malignancy from SVM models, it is possible to use probability scaling methods, such as the Vapnik scaling method, isotonic regression, or Platt scaling.

The SVM model, according to the invention, uses the Platt method, based on the criterion of the maximum likelihood method, to calculate the probability of malignancy of a sample.

Platt scaling transforms the results of the SVM classifier into a probabilistic model under which the data values are most likely. The evaluation of the probability of malignancy is based on training data.

The probability of malignancy of a tested sample is determined according to the criterion of maximum likelihood for a probabilistic model of the expression values of a set of 32 genes determined for this sample.

The set of 32 genes described in the subsequent sections was determined through research and microarray analyses.

In the first stage of the development of the expression classifier, the material was collected for the training set. The training set comprised 336 thyroid nodule samples, including 70 nodules with a cytologic diagnosis of a benign lesion (Bethesda II - B2), 68 nodules with a cytologic diagnosis of follicular lesion of undetermined significance (Bethesda III - B3), 61 nodules with cytologic diagnosis suspicious for a follicular neoplasm (Bethesda IV - B4), 47 nodules suspicious for thyroid cancer (Bethesda V -B5) and 90 nodules with a cytologic diagnosis of thyroid cancer (Bethesda VI - B6).

The clinical characteristics of the training set are presented in the table below.

| | **The whole training set** | **Benign nodules** | **Malignant nodules** |
|---|---|---|---|
| **Number** | **336** | **207** | **129** |
| **F/M** | **283/52** | **173/30** | **107/22** |
| **Median age** | **57** | | |
| **Symptoms** | **27/336** | **14/207** | **13/129** |
| **Median nodule diameter** | **15** | | |
| **EU TIRADS 3** | **99/328** | **78/201** | **21/127** |
| **EU TIRADS 4** | **102/328** | **81/201** | **21/127** |
| **EU TIRADS 5** | **127/328** | **42/201** | **85/127** |
| **Bethesda II** | **70/336** | **70/207** | **0/129** |
| **Bethesda III** | **68/336** | **65/207** | **3/129** |
| **Bethesda IV** | **61/336** | **56/207** | **5/129** |
| **Bethesda V** | **47/336** | **16/207** | **31/129** |
| **Bethesda VI** | **90/336** | **0/207** | **90/129** |

High-density microarray Human Transcriptome 2.0 (Affymetrix) was used to analyze the gene expression profile.

After preprocessing and background correction of the entire microarray set, the microarray set underwent a normalization process. To define the probe sets representing genes from the ENTREZ database, for all microarrays, a custom CDF (Chip Definition File) version 21 was used, which was downloaded from the website:
http://brainarray.mbni.med.umich.edu/Brainarray/Database/CustomCDF/CDF_download.asp The resulting data was further analyzed. A fusion of feature selection methods based on Fold Change and t-test, recursive SVM elimination (RFESVM), and RegVif [8] were used.

A radial kernel SVM classifier was used to validate the performance of the models. Classification quality was estimated with the bootstrap method, in which samples were drawn from the training set with replacement - 500 iterations. Feature selection, as well as classification and classification quality estimation, were carried out independently in each iteration for unbiased estimation of classifier models.

For variants and combinations of feature selection methods and classification algorithms, a bootstrap test of model classification quality was conducted. The medians of the individual quality indicators for the best model, along with the 95% confidence intervals, are summarized in the table below:

| | **accuracy** | **sensitivity** | **specificity** | **PPV** | **NPV** |
|---|---|---|---|---|---|
| Bootstrap (training set) | 0.925 [0.893 - 0.958] | 0.853 [0.769 - 0.929] | 0.972 [0.937- 1.000] | 0.946 [0.884 - 1.000] | 0.917 [0.870 - 0.961] |

Selection and classification with the best classification quality parameters were analyzed, and based on them, using the selection methods of choice on the whole training set, a set of 32 genes was determined for validation by RT-qPCR - Openarray - according to Table 1. Extended information on the set of genes is provided in Table 3.

**Table 1**

| L.p. | **Assay ID** | **Gene Symbol(s)** | **Gene Name(s)** | L.p. | **Assay ID** | **Gene Symbol(s)** | **Gene Name(s)** |
|---|---|---|---|---|---|---|---|
| 1 | Hs00611096_m1 | **AMOT** | angiomotin | 17 | Hs01120412_m1 | **KCNQ3** | potassium voltage-gated channel subfamily Q member 3 |
| 2 | Hs00163811_m1 | **C3** | complement component 3 | 18 | Hs01565584_m1 | **MET** | MET proto-oncogene, receptor tyrosine kinase |
| 3 | Hs00218591_m1 | **CAMK2N1** | calcium/calmodulin dependent protein kinase II inhibitor 1 | 19 | Hs00378551_m1 | **METTL7B** | methyltransferase like 7B |
| 4 | Hs00989715_m1 | **CFI** | complement factor I | 20 | Hs01083647_m1 | **MPZL2** | myelin protein zero like 2 |
| 5 | Hs00229255_m1 | **DCSTAMP** | dendrocyte expressed seven transmembrane protein | 21 | Hs01036720_m1 | **NOD1** | nucleotide binding oligomerization domain containing 1 |
| 6 | Hs00174944_m1 | **DIO1** | deiodinase, iodothyronine type I | 22 | Hs01552593_m1 | **OLR1** | oxidized low density lipoprotein receptor 1 |
| 7 | Hs00244839_m1 | **DUSP5** | dual specificity phosphatase 5 | 23 | Hs00153649_m1 | **PDE5A** | phosphodiesterase 5A |
| 8 | Hs00152928_m1 | **EGR1** | early growth response 1 | 24 | Hs00939867_m1 | **PSD3** | pleckstrin and Sec7 domain containing 3 |
| 9 | Hs00171315_m1 | **EMP2** | epithelial membrane protein 2 | 25 | Hs01120908_m1 | **SDC4** | syndecan 4 |
| 10 | Hs01034070_m1 | **FAM20A** | family with sequence similarity 20 member A | 26 | Hs01070627_m1 | **SLC26A4** | solute carrier family 26 member 4 |
| 11 | Hs00383314_m1 | **FAXC** | failed axon connections homolog | 27 | Hs01104163_m1 | **SLC26A7** | solute carrier family 26 member 7 |
| 12 | Hs01922255_s1 | **FLRT3** | fibronectin leucine rich transmembrane protein 3 | 28 | Hs00377618_m1 | **SLC5A8** | solute carrier family 5 member 8 |
| 13 | Hs00361131_g1 | **HBG1** | hemoglobin gamma-G | 29 | Hs00268204_m1 | **SLPI** | secretory leukocyte peptidase inhibitor |
| 14 | Hs00181853_m1 | **IGFBP6** | insulin like growth factor binding protein 6 | 30 | Hs01547334_m1 | **TM4SF1** | transmembrane 4 L six family member 1 |
| 15 | Hs00169943_m1 | **IGSF1** | immunoglobulin superfamily member 1 | 31 | Hs00892519_m1 | **TPO** | thyroid peroxidase |
| 16 | Hs00158127_m1 | **ITGA2** | integrin subunit alpha 2 | 32 | Hs00185147_m1 | **TUSC3** | tumor suppressor candidate 3 |

The developed SVM classifier has very good parameters useful for differentiating thyroid nodules. It is confirmed by the results of the study, in which 91% of the thyroid nodules examined were correctly classified as benign and malignant. The sensitivity and specificity of the diagnostic test were estimated at 83% and 96%, respectively.

The classification test includes a stage of sample normalization. An important element for proper validation of feature selection is the selection of reference genes. The essence of the analysis was to evaluate not only individual genes as candidates for reference genes but groups of genes that share these properties. Due to the importance of the correctness of the result, the normalization process is carried out using normalizing genes, characterized by stable expression levels between samples. It is assumed that each sample should show a certain minimum expression for at least four reference genes.

The calibrator samples are the biological material used to normalize the data between successive runs of the assay (successive arrays). It is assumed that each sample should show some minimum expression for at least four reference genes. It is claimed that in qPCR reaction a Ct below 28 indicates the sufficient signal of expressed gene.

The selection of reference genes was carried out using the iterative method of elimination of the least stable genes (NormFinder). The identified set of reference genes including EIF5, PPIE, UBE2D2, HADHA, EIF3A, and RPLPO is presented in Table 2.

**Table 2**

| **Lp** | **Gene name** | **ENTREZ ID** | **Assay ID** | **Amplicon sequence** | **3'.Most** | **Function** |
|---|---|---|---|---|---|---|
| **1** | **EIF3A (EIF3S10)** | 8661 | Hs01025769_m1 | aagctttcaa attctgcctc aatacacgcg taaggctgaa ttccgtaaact gtgtgacaa | **No** | Control gene |
| **2** | **HADHA** | 3030 | Hs00426191_m1 | gttgccatttcatgccaatacagaatagcaaca aaagacagaaaaacagtattaggta cccctgaagttttgctgggggccttacaggagca ggaggcacacaaaggctgcccaaaatggtgg gtgtgcctgctgtccatctgttctc | **No** | Control gene |
| **3** | **UBE2D2** | 7322 | Hs00366152_m1 | tgttgtgtgatcccaa tccagatgatcc tttagtgcctgagattgctcggatcta | **Yes** | Control gene |
| **4** | **EIF5** | 1983 | Hs01028813_g1 | aacgtatcccaccaaatattttggttgtgagctgg gagcacagacccagtttgatgttaagaatgac | **No** | Control gene |
| **5** | **PPIE** | 10450 | Hs01102333_m1 | gtctactatccatggccaactctggcccaaacac caatggctctcagttcttcctgacatgtgacaaga cagactggctggatggcaagcatgtg | **Yes** | Control gene |
| **6** | **RPLPO** | 6175 | Hs00420895_gH | aaactgctgcctcatatccggggggaatgtgggct ttgtgttcaccaaggaggacctcactgagatcag ggacatgt | **Yes** | Control gene |

Normalized gene expression levels are entered into the classifier.

In the next step, a material for the validation set was collected. RNA was isolated for selected samples from the validation set, and a quantitative PCR reaction and clinical analysis were performed.

The clinical analysis included 174 patients with a cytological diagnosis of the Bethesda II - B2, 119 with a diagnosis of the Bethesda III - B3, 126 with a diagnosis of the Bethesda IV - B4, 76 with a diagnosis of the Bethesda V - B5 category and 104 with a diagnosis of Bethesda VI - B6 category.

In B2 cytological category group, 10 patients were operated on, with no thyroid cancer diagnosed postoperatively. In B3 group, 55 patients were operated on, thyroid cancer was confirmed in 11 cases (1 patient - medullary thyroid cancer, 10 patients- follicular cancer). In B4 group, 85 patients were operated on. Thyroid cancer was diagnosed in 17 cases (4 patients - papillary thyroid carcinoma, 7 patients -follicular thyroid carcinoma, 3 patients - oxyphilic thyroid carcinoma, 3 patients - poorly differentiated carcinoma). In B5 group, 75 patients were operated on. Thyroid cancer was diagnosed in 63 cases (61 cases - papillary thyroid carcinoma, 2 cases - medullary thyroid carcinoma). In B6 group, all patients underwent surgical treatment, confirming cancer in 101 cases (99 cases - papillary carcinoma, 1 case - follicular carcinoma, 1 sample - poorly differentiated carcinoma).

The clinical analysis was followed by validation of the molecular classifier built based on the training set. To validate the classifier, 349 samples taken from 37 thyroid nodules classified after cytological evaluation as B2, 39 B3 nodules, 95 B4 nodules, 109 B5 nodules, and 69 B6 nodules were used.

Next, samples misclassified by the classifier were analyzed, finding no substantial errors and errors that may be due to the mistakenly assigned character of the sample. The predominant group of misclassifying lesions was small lesions, several millimeters in diameter, where it was likely that a second puncture dedicated to molecular evaluation missed the same lesion (25 samples). Another group of misclassified lesions originated from Hurthle-cells or were postoperatively diagnosed as follicular tumors of uncertain malignant potential (9 samples). In addition, for 17 samples, a potential cause of misclassification was not identified, which involved lesions defined as benign based on Bethesda II cytologic category or histopathologic evaluation.

The PCR classifier model, trained on a training set containing tumor samples with an assigned Bethesda II-VI category, is designed to predict tumor malignancy. For the target model, the cutoff threshold for the probability of malignant class is 0.35. Information related to the quality of the classifier and concerning the accuracy, sensitivity, and specificity are summarized in the table below:

| **Model name** | **Accuracy [95%Cl]** | **Sensitivity** | **Specificity** |
|---|---|---|---|
| **Training set (bootstrap)** | 0.872 [0.843 -0.903] | 0.792 | 0.936 |
| **Training set (resubstitution)** | 0.953 | 0.932 | 0.965 |
| **Independent validation set** | 0.862 | 0.775 | 0.953 |

The probability cutoff threshold makes it possible to control the sensitivity and specificity of the test, including the PPV (positive predictive value) and NPV (negative predictive value) parameters.

It was investigated how the quality parameters change when the threshold changes in the range of values 0.3-0.7 in the function of determining the class label. It is shown in Fig. 1. The classifier can be optimized against either PPV or NPV, and the results reveal the relationships between the different groups of parameters. Optimizing a classifier against PPV increases its specificity, and against NPV increases its sensitivity or accuracy. The highest classification accuracy is obtained when the threshold of the classifier function is as small as possible. In contrast, the highest PPV has a classifier with a high threshold for the classifier model but has lower classification accuracy and sensitivity. The highest NPV has a classifier for the lowest thresholds - classification accuracy is also highest for this setting.

In the context of diagnostic interpretation, where it is assumed that the positive class is malignant neoplasm - a high PPV indicates the probability of correct detection of malignant neoplasm. In contrast, a high NPV indicates the probability of correctly detecting a benign lesion (excluding a malignant lesion). Fig. 2 shows the classifier's quality characteristics as the ROC curve for the tested samples. The ROC curve shows the characteristics of the classifier's sensitivity and specificity. The shape of the ROC curve indicates that the classifier correctly labels benign / malignant.

The first step of the classifier is normalization using the set of reference genes listed in Table 2: EIF3A (EIF3S10), HADHA, UBE2D2, EIF5, PPIE, RPLPO.

For the classifier, the selected feature list contains 32 genes presented in Table 1, such as AMOT, C3, CAMK2N1, CFI, DCSTAMP, DIO1, DUSP5, EGR1, EMP2, FAM20A, FAXC, FLRT3, HBG1, IGFBP6, IGSF1, ITGA2, KCNQ3, MET, METTL7B, MPZL2, NOD1, OLR1, PDE5A, PSD3, SDC4, SLC26A4, SLC26A7, SLC5A8, SLPI, TM4SF1, TPO, and TUSC3, with normalized gene expressions as real numbers. The classifier displays on the computer screen the result of the classification test with information about the prediction of the class, the probability of malignancy, and the numerical values of all the features of the sample on the basis of which the classification took place. In addition, the classifier displays boxplots or violin plots for the benign and malignant classes of each gene in the training population with the expression value of the test sample plotted.

The performance of the classifier was confirmed in an experimental setting, where samples of thyroid nodules with an established malignant result were validated in a post-operative histopathological examination, while benign nodules were validated in the long-term follow-up of patients.

The study used total RNA isolated from material from a fine-needle biopsy of thyroid nodules taken under ultrasound guidance.

The methods for RNA extraction are well known in the field and described in standard molecular biology textbooks. Such an exemplary procedure for processing a biological sample, including isolation of the total RNA of a cell, is described in Example 1, which illustrates one of the possible implementations of the invention.

Gene expression levels can be determined by quantitative RT-PCR (quantitative reverse transcription PCR, RT-qPCR) or other PCR-based methods, as well as microarray-based methods or NGS transcriptome sequencing, or others known in the field or combinations thereof.

In order to determine the suitability of the method according to the invention for evaluating nodule samples with significantly different RNA content - two subsets were extracted from the validation set samples depending on the measured RNA concentration. The characteristics of the sets are shown in the table below:

| **Set** | **Sample number** | **Number of malignant samples** |
|---|---|---|
| Samples containing RNA at a concentration of <20ng/ml | 194 | 91 |
| Samples containing RNA at a concentration ≥ 20ng/ml | 128 | 73 |

Measurement of gene expression was carried out according to the established rules taking into account different RNA concentrations of RNA. Quality parameters for classification were calculated:

| **RNA concentration** | **Accuracy** | **Sensitivity** | **Specificity** | **PPV** | **NPV** |
|---|---|---|---|---|---|
| <20ng/ml | 0.851 | 0.703 | 0.981 | 0.970 | 0.789 |
| 20ng/ml | 0.883 | 0.863 | 0.909 | 0.926 | 0.833 |

Classification quality parameters for both sets take high values, indicating that the described method can be implemented to the full range of thyroid nodule samples containing RNA at high as well as low concentrations.

### Example 1

Sample preparation and classification of a sample with uncertain cytological evaluation.

A fine-needle biopsy of the nodule was performed in a patient with a suspected thyroid nodule. According to the TBSRTC (The Bethesda System for Reporting Thyroid Cytology), the material was assigned to category IV, suspicious for a follicular neoplasm, which may warrant surgery.

Total RNA isolated from a fine-needle biopsy material was used in this analysis. RNA isolation was performed using Qiagen's miRNeasy micro kit according to the manufacturer's recommendations. RNA concentration was measured with the high-sensitivity Qubit^{™} RNA HS Assay Kit using a Qubit 2.0 fluorometer. 20 ng of total RNA was used for the reaction.

For cDNA synthesis, a Sensiscript cDNA reagent kit was used, with Sensiscript Reverse Transcriptase (RT-S), RT buffer, dNTPS mixture, and RNase-free water. Random Nanomers, OligodT(23) primers, and RNase inhibitor at 40 units/µl were also used. The initial RT buffer was diluted to make 1x concentrated buffer, with which the inhibitor was diluted in a 3:1 ratio.

The composition of the cDNA synthesis is summarized in the table below:

| Reagents | Volume (per reaction) | Final concentration |
|---|---|---|
| 10x Buffer RT | 1 µl | 1x |
| dNTP (5nM each) | 1 µl | 0,5 nM each |
| Random Nanomer Primers (50 µM) | 0,8 µl | 2,5 µM |
| OligodT(n23) Primer (40 µM) | 0,25 µl | 1 µM |
| RNAse Inhibitor (10 units/µl) | 0,5 µl | 1 unit |
| RT-S Polymerase (4 units/µl) | 0,5 µl | 0,2 unit |
| RNAse free H₂O | 5,95 µl | |
| Final volume per sample | 10 µl | |

An additional pre-amplification step (pre-amplification) is used for RNA samples with low RNA concentrations, allowing real-time PCR reaction without introducing amplification bias. All samples from fine-needle biopsies of thyroid nodules were preamplified.

The pre-amplification step was carried out using TaqMan PreAmp Master Mix reaction mixture and Taqman Preamp Pool gene-specific primer mix. The pre-amplification reaction was carried out using a QuantStudio 12k Flex system in 96-well plates under the following conditions:
- AmpliTaq Gold DNA polymerase activation - 10 minutes, 95°C
- denaturation - 15 seconds, 95°C
- attachment of primers and chain elongation - 1 minute, 60°C.
- completion of the reaction - 10 minutes, 99°C
   14 cycles of amplification were performed. After the reaction, the sample was diluted 20x by adding 190 ul of RNase-free water.

The composition of the cDNA pre-amplification reaction is summarized in table below:

| Reagents | Volume (per reaction) | Final concentration |
|---|---|---|
| 2x Taqman Preamp Master mix | 5 µl | 1x |
| Taqman Preamp Pool | 2,5 µl | |
| cDNA template | 2,5 µl | |
| Final volume | 10 µl | |

In the next step, the reaction mixture, RNase-free water, and preamplified cDNA were applied to the 384-well plate. The plate was centrifuged for 1 min at 2,000 rpm. Next, samples were transferred from the 384-well plate to the OpenArray at the dispensing station. The OpenArray plate was sealed with foil and placed in the press for 10s. After removal, the plate was filled with oil, and the cap was sealed.

The composition of the cDNA amplification reaction is summarized in the table below:

| Reagents | Volume (per reaction) |
|---|---|
| 2x TaqmanOpenarray Real-Time PCR | |
| Mastermix | 2,6 µl |
| RNAse-free water | 1,4 µl |
| cDNA template | 1,5 µl |
| Final volume | 5,5 µl |

The qPCR reaction was performed on a QuantStudio 12K FLEX system with an OpenArray block according to the Openarray Taqman Gene Expression protocol (Thermofisher).

To minimize errors and the impact of variations between samples, the qPCR reaction was performed using reference RNAs. Normalization of the expression measured on the qPCR plate was carried out using a calibrator and six reference genes (EIF5, PPIE, UBE2D2, HADHA, EIF3A (EIF3S10), RPLPO). The result was analyzed, including whether all reference genes returned a signal from the quantitative RT-PCR reaction. The condition for correct normalization is that all reference genes return a signal.

The steps described above refer to the evaluation of gene expression in the analyzed sample. The normalized gene expression values are entered into the classifier to obtain information about the nature of the thyroid nodule.

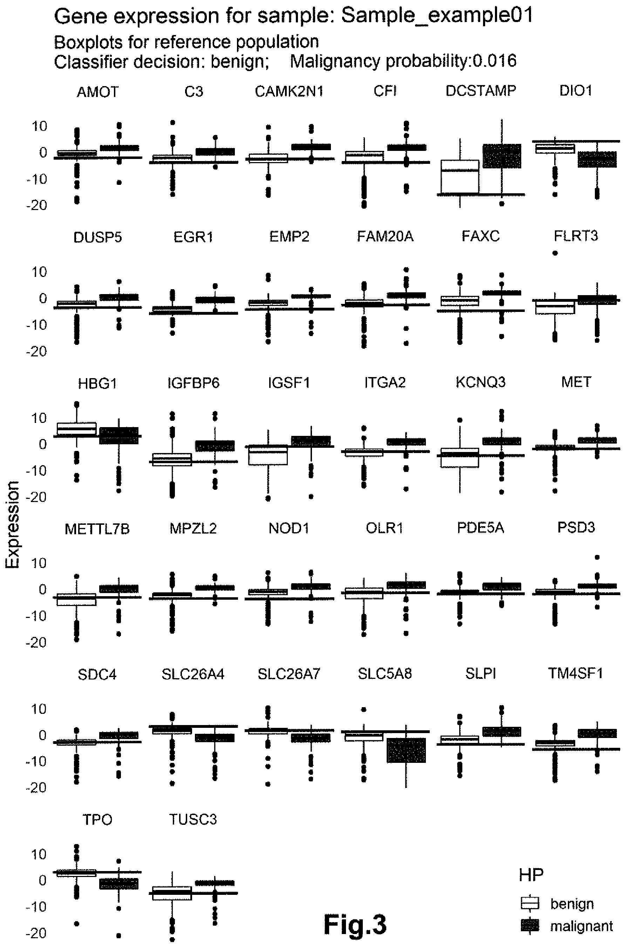

Fig.3 shows the expression of individual genes. The values for the test sample are marked with a horizontal line against the population used to teach the model.

Considering the expression of the entire gene set, the sample was classified as benign, with a probability of a malignant lesion of 0.016 (1.6%). Further observation of the patient confirmed the nature of the examined nodule as benign.

### Example 2

A fine-needle biopsy of the nodule was performed in a patient with a suspected thyroid nodule. According to TBSRTC, the specimen was assigned to category IV (suspicious for a follicular neoplasm), which may warrant surgery.

The analysis of the expression of a gene set on the plate by RT-qPCR array was performed, followed by the normalization and finally classification of the sample.

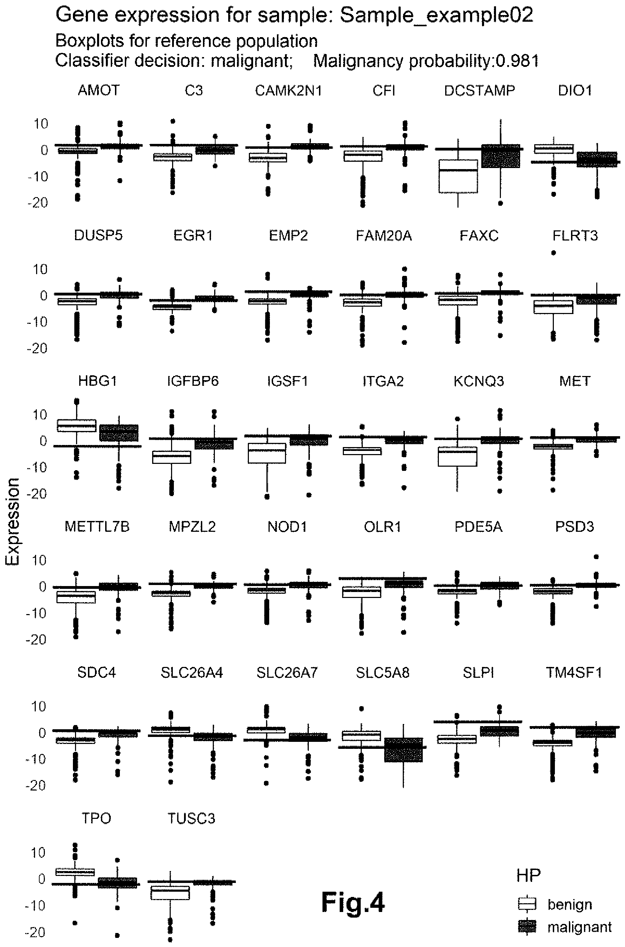

The expression of individual signature genes for the test sample, shown in the graphs in Fig.4, is indicated by a horizontal line against the population used to train the model. Taking into account the expression of the entire set of genes, the sample was classified as malignant, with a probability of a malignant lesion of 0. 981 (98.1%). The postoperative evaluation confirmed the nature of the test lesion as malignant.

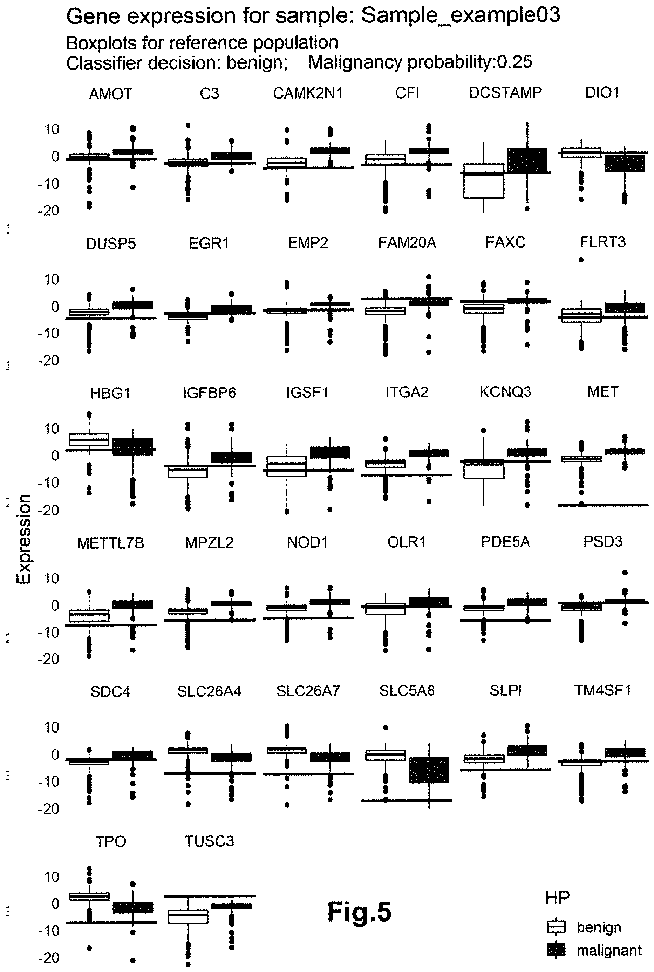

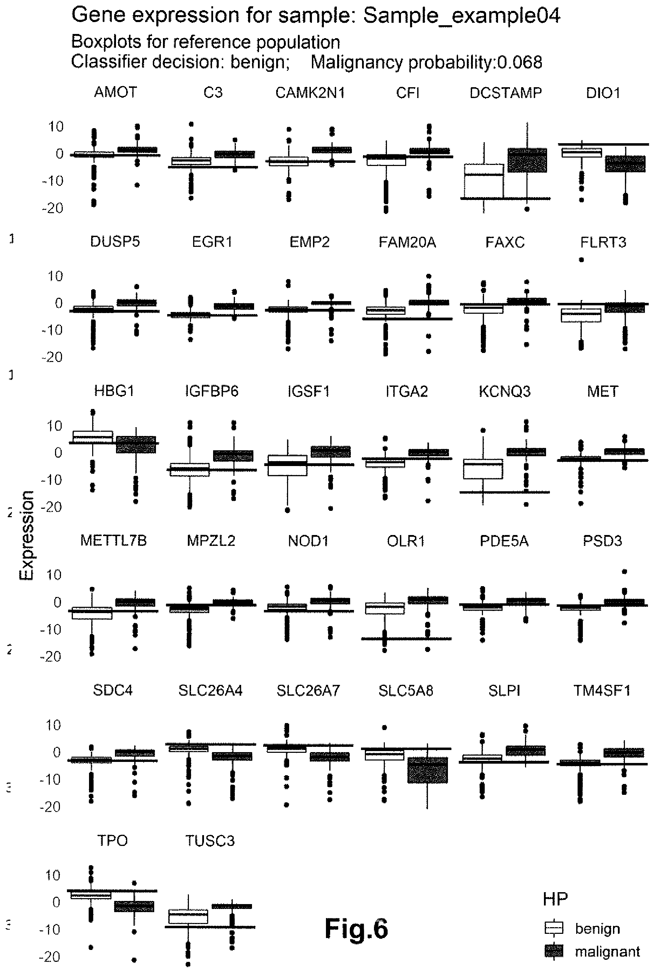

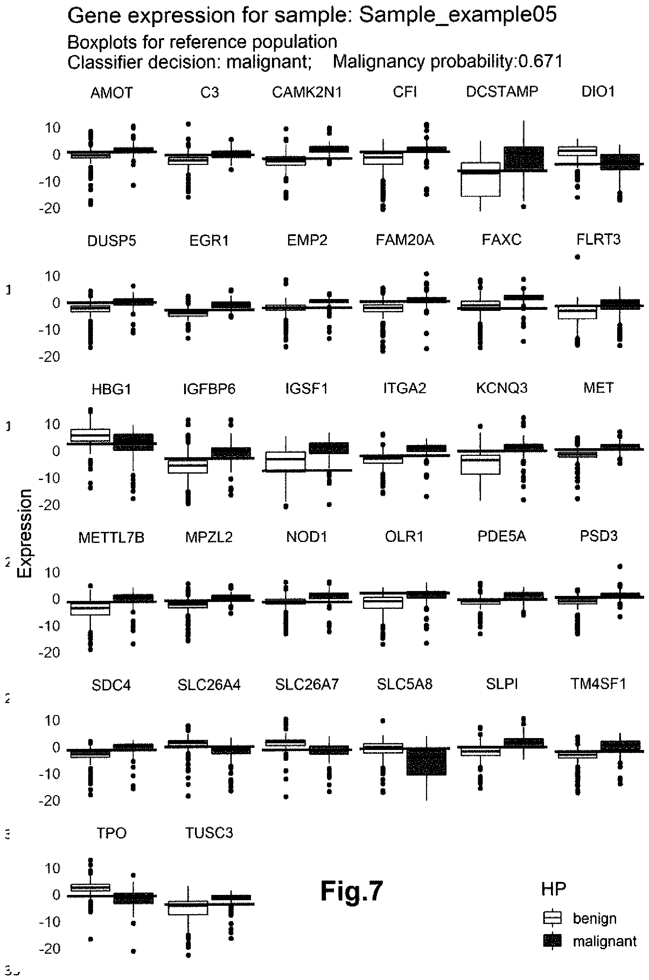

Further examples of determining the nature of lesions in fine-needle biopsy samples of thyroid nodules using the expression of the selected 32 genes are shown in Fig. 5, Fig. 6, and Fig.7. Considering the expression of the set of selected 32 genes, two samples of thyroid nodules (Fig. 5 and Fig. 6) were classified as benign, with a probability of a malignant lesion of 0.251 (25.1%) and 0.069 (about 7%), respectively. In contrast, the third thyroid nodule sample (Fig. 7) was classified as malignant, with a probability of malignancy of 0.69 (69%).

### References:

1.Alexander, Erik K, Giulia C Kennedy, Zubair W Baloch, Edmund S Cibas, Darya Chudova, James Diggans, Lyssa Friedman, et al. 2012. "Preoperative Diagnosis of Benign Thyroid Nodules with Indeterminate Cytology." The New England Journal of Medicine 367 (8): 705-15. https://doi.org/10.1056/NEJMoa1203208.
2.Chudova, Darya, Jonathan I Wilde, Eric T Wang, Hui Wang, Nusrat Rabbee, Camila M Egidio, Jessica Reynolds, et al. 2010. "Molecular Classification of Thyroid Nodules Using High-Dimensionality Genomic Data." The Journal of Clinical Endocrinology and Metabolism 95 (12): 5296-5304. https://doi.org/10.1210/jc.2010-1087.
2.González, Hernán E., José R. Martinez, Sergio Vargas-Salas, Antonieta Solar, Loreto Veliz, Francisco Cruz, Tatiana Arias, et al. 2017. "A 10-Gene Classifier for Indeterminate Thyroid Nodules: Development and Multicenter Accuracy Study." Thyroid 27 (8): 1058-67. https://doi.org/10.1089/thy.2017.0067.
4.Lithwick-Yanai, Gila, Nir Dromi, Alexander Shtabsky, Sara Morgenstern, Yulia Strenov, Meora Feinmesser, Vladimir Kravtsov, et al. 2017. "Multicentre Validation of a MicroRNA-Based Assay for Diagnosing Indeterminate Thyroid Nodules Utilising Fine Needle Aspirate Smears." Journal of Clinical Pathology 70 (6): 500-507. https://doi.org/10.1136/jclinpath-2016-204089.
5.Nikiforov, Yuri E., Sally E. Carty, Simon I. Chiosea, Christopher Coyne, Umamaheswar Duvvuri, Robert L. Ferris, William E. Gooding, et al. 2015. "Impact of the Multi-Gene ThyroSeq Next-Generation Sequencing Assay on Cancer Diagnosis in Thyroid Nodules with Atypia of Undetermined Significance/Follicular Lesion of Undetermined Significance Cytology." Thyroid 25 (11): 1217-23. https://doi.org/10.1089/thy.2015.0305.
6.Zafereo, Mark, Bryan Mclver, Sergio Vargas-Salas, José Miguel Dominguez, David L. Steward, F. Christopher Holsinger, Emad Kandil, et al. 2020. "A Thyroid Genetic Classifier Correctly Predicts Benign Nodules with Indeterminate Cytology: Two Independent, Multicenter, Prospective Validation Trials." Thyroid 30 (5): 704-12. https://doi.org/10.1089/thv.2019.0490.
7.Haugen B, Alexander E, Bible KC, Doherty GM, Mandel SJ,.Nikiforov YE, Pacini F I wsp. 2015 American Thyroid Association Management Guidelines for Adult Patients with Thyroid Nodules and Differentiated Thyroid Cancer: The American Thyroid Association Guidelines Task Force on Thyroid Nodules and Differentiated Thyroid Cancer. Thyroid. 2016 Jan 1; 26(1): 1-133 8.Cibas ES, Ali SZ. The 2017 Bethesda System for Reporting Thyroid Cytopathology. Thyroid. 2017 Nov;27(11):1341-1346
9. Ke Zhang, Jiangiu Liu, Cuilin Li, Xiaowei Peng, Hui Li, Zhi Li, Identification and validation of potential target genes in papillary thyroid cancer; European Journal of Pharmacology vol.843 (2019), pages 217-225,XP085569568, ISSN:0014-2999, doi.org/10.1016/j.ejphar.2018.11.026
   10. Student S., Pluciennik A., Jakubczak M., Fujarewicz K. (2018) Feature Selection Based on Logistic Regression for 2-Class Classification of Multidimensional Molecular Data. In: Agre G., van Genabith J., Declerck T. (eds) Artificial Intelligence: Methodology, Systems, and Applications. AIMSA 2018. Lecture Notes in Computer Science, vol 11089. Springer, Cham. https://doi.org/10.1007/978-3-319-99344-7_29

**Table 3.**

| **Assay.ID** | **Gene Symbol(s)** | **Gene Name(s)** | **Gene Alias(es)** | **RefSeq(s)** | **GenBank mRNA(s)** | **Amplicon sequence** |
|---|---|---|---|---|---|---|
| Hs00611096_m1 | **AMOT** | angiomotin | | XM_011530875.2;XM_005262090.1; NM_001113490.1;XM_005262087.1; NM_133265.2;XM_017029289.1 | AB028994.3;AF286598.1; AY987378.1;BC130294.1 | agaggattctggctctggaagctgatatgac aaagtgggagcagaaatatttggaggag aatgtg |
| Hs00163811_m1 | **C3** | complement component 3 | AHUSS;ARMD9;ASP; C3a; C3b;CPAMD1; HEL-S-62p | NM_000064.3 | K02765.1;BC150179.1;EU794602.1; BC150200.1;BP201387.1 | aatgtg ctgtgtggcagacccccttcgaggtcacagtaatgc aggacttcttcatcgacctgcggctaccctactctg ttgttcgaaacgagcag |
| Hs00218591_m1 | **CAMK2N1** | calcium/calmodulin dependent protein kinase II inhibitor 1 | PRO1489 | NM_018584.5 | AY204901.1 | cgggttgttattgaagatgataggattgatgacgtgct gaaaaatatgaccgacaaggcacct |
| Hs00989715_m1 | **CFI** | complement factor I | AHUS3;ARMD13; C3BINA;C3b-INA; FI; IF;KAF | XM_006714210.3;XM_006714209.1; XM_017008164.1;XM_017008165.1; XM_017008166.1;XM_005262976.1; XM_011531920.1;NM_000204.4; NM_001318057.1 | AK122686.1;Y00318.1; AK290625.1; J02770.1; AK299232.1 | ctcagagccagtaaaactcatcgttaccaaatat ggacaacagtagtagactggatacaccccg accttaaacgt |
| Hs00229255_m1 | **DCSTAMP** | dendrocyte expressed seven transmembrane protein | FIND;TM7SF4; hDC-STAMP | XM_017013878.1;XM_011517321.1;NM_030788.3 | BC112020.1;AK095417.1; BC069349.1;BC112018.1; AF277290.1;AF305068.1 | aggaactcaatattatccatgattcttcctttaatata tctgtgtttgaacccaactgtatcccaaaaccaaa attccttctatctgagacctgggttcctctc |
| Hs00174944_m1 | **DIO1** | deiodinase, iodothyronine type I | 5DI;TXDI1 | NR_136693.1;NM_000792.6; NR_136692.1;NM_001039715.2; NM_213593.4_ | AY560383.1;AY560374.1;AK290780.1; AY560382.1;AY560377.1;AY560375.1; BC107170.2;AY560379.1;BC1 07171.2; CD014030.1; AY560380.1;FJ002243.1 | gtaaatctcccttggaactacaacccagagg aagttcgtgctgttctggaaaagctccacagttaa tctggacagatacctcaattctaggtga |
| Hs00244839_m1 | **DUSP5** | dual specificity phosphatase 5 | DUSP;HVH3 | NM_004419.3 | BC062545.1; U16996.1 | aggggatatgagactttctactcggaatat cctgagtgttgcgtggatgtaaaacccatttca caagagaagattgagagtgagagag ccctcatcagccagtgtg |
| Hs00152928_m1 | **EGR1** | early growth response 1 | AT225;G0S30; KROX-24;NGFI-A; TIS8;ZIF-268; ZNF225 | NM_001964.2 | KM114058.1; AK298101.1;X52541.1; M62829.1;BC073983.1; AK301065.1; M80583.1 | cttttcctgacatctctctgaacaacgagaaggtgct ggtggagaccagttaccccagccaaaccactcgac |
| Hs00171315_m1 | **EMP2** | epithelial membrane protein 2 | XMP | NM_001424.5; XM_006720864.3 | U52100.1;AK313134.1 ;BF981157.1; BC009687.1;X94770.1 | agagtactccacgctgcaggcggtccaggccacc atgatcctctccaccattctctgctgcatcgccttctt catcttcgtgctccag |
| Hs01034070_m1 | **FAM20A** | family with sequence similarity 20 member A | AI1NG,AIGFS;FP2747 | NM_001243746.1;XM_011524918.2; XM_006721959.2;XM_017024781.1; NM_017565.3 | BC136686.1;AL133105.1; AB545606.1 ;AK298071.1;AK056789.1 ;AY358197.1; BC136689.1 | aggcactttcccaccatcagtgctgattacagtc aagatgagaaagccttgctgggggcatgtgact |
| Hs00383314_m1 | **FAXC** | failed axon connections homolog | C6orf168;dJ273F20 | XM_006715582.2;XM_011536188.2; NM_032511.2 | BC011583.1;AK057793.1;BC004869.2; AK096480.1; BC006515.2 | caaaggtgagctgatcaaccttgccatgtactgtgag aggataaggaggaaattttggccagagtggcac |
| Hs01922255_s1 | **FLRT3** | fibronectin leucine rich trans membrane protein 3 | HH21 | XM_011529205.2;NM_198391.2; NM_013281.3;XM_005260682.4; XM_011529204.2 | AB040902.1;AK074909.1; AK027670.1 | caattttccagatattatataatgaactcgtaatgt aacatttccttctagcttcctactgaattgtgagct gttacttgttgaaaaaccatatcacttttctgttg ccatgatttttttttttcaacaaaaaacca aagtgcattgtacgccc |
| Hs00361131_g1 | **HBG1** | hemoglobin gamma-G | HBGA; HBGR; HSGGL1;PR02979 | NM_000559.2;NM_000559.3 | BC010913.1 BC020719.1 | cctggttgtctacccatggacccagaggttctttg acagctttggcaacctgtcctctg |
| Hs00181853_m1 | **IGFBP6** | insulin like growth factor binding protein 6 | IBP6 | NM_002178.2 | AK313158.1;BC010162.2; M69054.1;BC011708.2; BQ925731.1;M62402.1; BC003507.1;CR541791.1; BC005007.1 | gctgttgcagaggagaatcctaaggagagtaaa ccccaagcaggcactgcccgcccacaggatgtg aaccgcagagaccaacagaggaatccagg cacctctaccacgccctcccag cccaattctgcgggtgtccaagacactg |
| Hs00169943_m1 | **IGSF1** | immunoglobulin superfamily member 1 | CHTE;IGCD1 ; IGDC1;INHBP; PGSF2;p120 | NM_001170963.1;NM_001170962.1; NM_001555.4;XM 011531330.1; NM_205833.3;XM_011531334.1; NM_001170961,1,XM_011531333.1 | Y10523.1;BC063884.1; AK057916.1;AF034198.1; AK226008.1;AK307871.1; AB058894.2 | gccaactgcccaagcccatcttctggatt caggctgaga cccccgctcttcct gggtgtaatgttaacatcctctgcc |
| Hs00158127_m1 | **ITGA2** | integrin subunit alpha 2 | BR;CD49B;GPIa; HPA-5;VLA-2; VLAA2 | NM_002203.3;NR_073103.1; NR_073104.1;NR_073105.1; NR_073106.1 | BC143509.1;AK307952.1; BC143508.1;BP280760.1; X17033.1;BC143505.1; BC143511.1 | attttaaattgttgtttggcctacaatgttggtct cccagaagcaaaaatattttccggtccttcaa |
| Hs01120412_m1 | **KCNQ3** | potassium voltage-gated channel subfamily Q member 3 | BFNC2; EBN2; KV7.3 | XM_006716555.3;XM_017013400.1 ; NM_004519.3;NM_001204824.1; XM_005250914.3;XM_011517026.2 | AK296293.1;AF033347.1; BC128576.1 | aaagaactcatcacggcctggtacatcggttt cctgacactcatcctttcttcat |
| Hs01565584_m1 | **MET** | MET proto-oncogene, receptor tyrosine kinase | AUTS9; DFNB97; HGFR;RCCP2; c-Met | XM_006715990.2; M_001127500.2; NM_001324401.1; M_001324402.1; XM_011516223.1 ;NM_0002453 | EU826572.1;EU826573.1; EU826575.1; AB209898.1;EU826574.1;X54559.1; EU8265761;J02958.1;EU176015.1; EU826567.1; BC130420.1;EU826577.1; EU826568.1;EU826569.1;EU826570.1; EU826579.1;EU826571.1 ;AK296974.1 | ggttgtggtttctcgatcaggaccatcaacccct atgtgaattttctcctggactcccatccagtgtctcca |
| Hs00378551_m1 | **METTL7B** | methyltransferase metyiransierase like 7B | ALDI | NM_152637.2 | AY358508.1;AK290112.1 | agaccgggaggtgtgctctttttct gggagcatgt ggcagaaccatatggaagctgggccttcatgtgg cagcaagttttcg |
| Hs01083647_m1 | **MPZL2** | myelin protein zero like 2 | EVA; EVA1 | NM_144765.2; NM_005797.3 | AY359061.1;BC017774.1; AK290326.1;DA163069.1; AF275945.1;AF030455.1 AF304447.1 | ctctttggcctatagcagctgtggaaatttatacct cccgggtgctggaggctgttaatgggacaga tgctcggttaagctg |
| Hs01036720_m1 | **NOD1** | nucleotide binding oligomerization domain containing 1 | CARD4;CLR7.1; NLRC1 | XM_011515079.1;XM_005249572.1; NM_006092.2;XM_005249568.1; XM_011515088.2;XM_011515087.1; XM_017011674.1;XM_017011675.1; XM_011515085.1;XM_011515084.1; XM_011515083.1;XM_006715633.2; XM_011515081.2;XM_005249576.1; XM011515080.2 | AF126484.1;AK023969.1; AF113925.1;BC040339.1; KU178514.1 | tgctcggttaaaatg cagactcagcgtaaaccagatcactgacggt ggggtaaaggtgctaagcgaagag ctgaccaaatac |
| Hs01552593_m1 | **OLR1** | oxidized low density lipoprotein receptor 1 | CLEC8A;LOX1; LOXIN;SCARE1 ;SLOX1 | NM_001172633.1; NM_001172632.1 NM_002543.3 | AF035776.1; BC022295.1;AK295409.1; AK292124.1; AB010710.1;AK308009.1; AL551100.3;AB102861.1;AX429248.1; BP219342.1 | taaaggtcttcagtttctttactctccatggtggt gcctggctgctgcgactctaggggtcctttgcc |
| Hs00153649_m1 | **PDE5A** | phosphodiesterase 5A | CGB-PDE;CN5A; PDE5 | NM_001083.3; NM_033437.3; NM_033430.2; M_017008791.1 | AK290189.1;AY266363.1;JQ286346.1 ;BC126233.1;AB015656.1;AJ004865.1; AY264918.1;AF043731.1 | ttggtgtagcccaggccatcaacaagaaatc aggaaacggtgggacatttactgaaaaagat gaaaaggactttgctgcttat |
| Hs00939867_m1 | **PSD3** | pleckstrin and Sec7 domain containing 3 pleckstrin i Sec7 | EFA6D;EFA6R; HCA67 | XM_011544468.2;NM_015310.3; XM_011544473.2;XM_017013265.1; XM_011544469.2;XM_017013264.1; XM_011544467.2;XM_017013263.1; NM_206909.2;XM_011544471.2; XM_017013258.1;XM_017013262.1; XM_017013261.1;XM_017013260.1; XM_017013259.1 | GU727648.1;AK315099.1; AK091317.1;DB055312.1; AF243495.2;AF519767.1; AL832778.1; BC075045.2; BC075044 2; AB023159.2 | caggtatttctttaaagcattctctcttgtggg agaaactcaagaacgagagagagagttttaa tacacttctccaatagatatt |
| Hs01120908_m1 | **SDC4** | syndecan 4 | SYND4 | NM_002999.3; XM_011528977.2 | CR542074.1;X67016.1;BC030805.1; AK223243.1;AK303964.1;AK222695.1; D13292.1;AK312507.1;CR542045.1 | tggtgcctctagataaccatatccctgagaggg cagggtct gggagccaagtccccaccgaaccc aagaaactagaggagaatgaggttatccccaa gagaatctcacccgttgaagagagtgaggatgt gtccaacaaggtgtcaa |
| Hs01070627_m1 | **SLC26A4** | solute carrier family 26 member 4 | DFNB4;EVA; PDS;TDH2B | XM_005250425.2; M_000441.1; XM_006716025.3;XM_017012318.1 | AF030880.1 | aagggatggcatatgccctactagctgcagttcct gtcggatatggtctctactctgctttttccctatcc |
| Hs01104163_m1 | **SLC26A7** | solute carrier family 26 member 7 | SUT2 | NM_052832.3; NM_001282357.1; NM_001282356.1; NM_134266.1 | AJ413229.2; AF331521.1; BC114474.1;AJ413228.1; AJ413230.1;BC113866.1; AK122933.1 | gttgaggtttacatggactgtaaaggcagga gtgtggatgtattgttagcccattgtacagctt ccttgataaaagcaatgacgt |
| Hs00377618_m1 | **SLC5A8** | solute carrier family 5 member 8 | AIT;SMCT;SMCT1 | XM_017018910.1; NM_145913.3 | AY081220.1; AF536216.1; AK313788.1 ;AK075263.1; BC110492.2 | catgccttatttggtactggacattctgcaagatt atccaggacttcctggactttttgtggcctgtg cttacagtgggacattaagc |
| Hs00268204_m1 | **SLPI** | secretory leukocyte peptidase inhibitor | ALK1,ALP;BLPI; HUSI;HUSI-I;MPI; WAP4;WFDC4 | NM_003064.3 | X04470.1; AF114471.1; AX772818.1;AK312192.1; BC020708.1;X04503.1 | ttcaaagctggagtctgtcctcctaa gaaatctgcccagtgccttagatacaag aaacctgagtgccag |
| Hs01547334_m1 | **TM4SF1** | transmembrane 4 L six family member 1 | H-L6;L6;M3S1; TAAL6 | XM_017006385.1; NM_014220.2 | BC010166.2;AK130073.1; BC034145.1;CR456953.1; BC008442.1;M90657.1; AK093829.1;AK130271.1 | ccttctggatacctccacatggtccgag tgcactgaacccaagcacattgtggaat ggaatgtatctct |
| Hs00892519_m1 | **TPO** | thyroid peroxidase | MSA;TDH2A;TPX | NM_175719.3;NM_175721.3; NM_175722.3;NM_001206744.1 ; NM_001206745.1;NM_000547.5; XM_011510380.2;XM_011510381.2; XM_111510379.2 | AB208960.1;X17358.1; AF533528.1;J02970.1; AF439430.1;Y00406.1; DA958481.1;M17755.2; BC095448.1;J02969.1 | gaccaccccagatgggg cgcctccaacacggccctgg cacgatggctccctcc |
| Hs00185147_m1 | **TUSC3** | tumor suppressor candidate 3 | D8S1992;M33; MRT22;MRT7; N33;OST3A | XM_011544651.2; NM_006765.3; XM_017013861.1; NM_178234.2; XM_011544654.2; XM_017013860.1; XM_011544652.2 | U42349.1; BT020002.1; BC010370.1 | ccgctatcaccatgggggatggttcttctaaatgaa gcagcaacttcgaaaggcgatgttggaaaaag acggataatttgcctagtgggattgggcc |

## Claims

1. A method of differentiating benign and malignant thyroid nodules in tumor biopsy samples, including:
- RNA isolation steps, and
- determining of expression levels of genes and using a classifier
- comparing the gene expression of the thyroid nodule sample with the gene expression of the thyroid nodule samples in the training set, and then
- identifying the nature of the nodule as malignant or benign and determining the probability of malignancy of the thyroid nodule,
with the classifier trained on gene expression data correlated with the nature of the thyroid nodule, especially benign thyroid nodules and malignant tumors, including papillary and follicular thyroid carcinoma,
**characterized in that**
the expression level of a set of 32 genes is determined, which consists of AMOT, C3, CAMK2N1, CFI, DCSTAMP, DIO1, DUSP5, EGR1, EMP2, FAM20A, FAXC, FLRT3, HBG1, IGFBP6, IGSF1, ITGA2, KCNQ3, MET, METTL7B, MPZL2, NOD1, OLR1, PDE5A, PSD3, SDC4, SLC26A4, SLC26A7, SLC5A8, SLPI, TM4SF1, TPO, and TUSC3,
and the normalization of the expression of each gene of this set is carried out under the endogenous control of the following six normalizing genes EIF3A (EIF3S10), HADHA, UBE2D2, EIF5, PPIE, and RPLPO,
and the malignant or benign nature of the nodule sample is determined with at least 91% specificity and at least 83% classification sensitivity for the normalized expression level of a set of 32 genes.

2. The method, according to claim 1, **characterized in that** the nature of the nodule and the malignancy probability of the tested sample is determined according to the decision rule of the classifier, taking into account the expression level data of the set of 32 genes and the gene expression level of each sample of the nodule in the training set, determined for each sample of the training set for the same set of 32 genes and under the same normalization conditions.

3. The method according to claim. 2, **characterized in that** the nature and probability of malignancy of a nodule sample are determined according to the criterion of maximum likelihood for a probabilistic model of the normalized expression values of a set of 32 genes of the sample.

4. The method according to claim. 1, **characterized in that** RT-qPCR, oligonucleotide microarrays, or RNA-seq transcriptome sequencing are used to detect the expression level of a set of genes.

5. Use of the set of hybridization probes Hs00611096_m1, Hs00163811_m1, Hs00218591_m1, Hs00989715_m1, Hs00229255_m1, Hs00174944_m1, Hs00244839_m1, Hs00152928_m1, Hs00171315_m1, Hs01034070_m1, Hs00383314_m1, Hs01922255_s1, Hs00361131_g1, Hs00181853_m1, Hs00169943_m1, Hs00158127_m1, Hs01120412_m1, Hs01565584_m1, Hs00378551_m1, Hs01083647_m1, Hs01036720_m1, Hs01552593_m1, Hs00153649_m1, Hs00939867_m1, Hs01120908_m1, Hs01070627_m1, Hs01104163_m1, Hs00377618_m1, Hs00268204_m1, Hs01547334_m1, Hs00892519_m1, Hs00185147_m1 for detecting in a thyroid nodule sample the expression level of a corresponding gene from a set of 32 genes AMOT, C3, CAMK2N1, CFI, DCSTAMP, DIO1, DUSP5, EGR1, EMP2, FAM20A, FAXC, FLRT3, HBG1, IGFBP6, IGSF1, ITGA2, KCNQ3, MET, METTL7B, MPZL2, NOD1, OLR1, PDE5A, PSD3, SDC4, SLC26A4, SLC26A7, SLC5A8, SLPI, TM4SF1, TPO, TUSC3 and for determining the nature of the nodule by the method according to claims. 1 to 3, using a computer software product having a computer-readable medium and resources for measuring the normalized expression level of a set of genes, performing classification, and determining the probability of malignancy of a nodule sample.

## Patentansprüche

1. Verfahren zur Unterscheidung zwischen benignen und malignen Schilddrüsenknoten in Tumorbiopsieproben, umfassend:
- Schritte zur RNA-Isolierung, und
- Bestimmung der Expressionsniveaus von Genen sowie Verwendung eines Klassifikators
- Vergleich der Genexpression der Schilddrüsenknotenprobe mit der Genexpression der Schilddrüsenknotenproben aus dem Trainingsdatensatz, und anschließend
- Identifizierung der Natur des Knotens als malign oder benignund Bestimmung der Wahrscheinlichkeit der Malignität des Schilddrüsenknotens,
mit einem Klassifikationsalgorithmus, der auf Genexpressionsdaten trainiert wurde, die mit der Natur des Schilddrüsenknotens korrelieren, insbesondere benignen Schilddrüsenknoten und malignen Tumoren, einschließlich papillärem und follikulärem Schilddrüsenkarzinom,
wobei
das Expressionsniveau eines Satzes von 32 Genen bestimmt wird, der aus AMOT, C3, CAMK2N1, CFI, DCSTAMP, DIO1, DUSP5, EGR1, EMP2, FAM20A, FAXC, FLRT3, HBG1, IGFBP6, IGSF1, ITGA2, KCNQ3, MET, METTL7B, MPZL2, NOD1, OLR1, PDE5A, PSD3, SDC4, SLC26A4, SLC26A7, SLC5A8, SLPI, TM4SF1, TPO und TUSC3 besteht,
und die Normalisierung der Expression jedes Gens dieses Satzes unter der endogenen Kontrolle der folgenden sechs Normalisierungsgene erfolgt: EIF3A (EIF3S10), HADHA, UBE2D2, EIF5, PPIE und RPLPO,
und die maligne oder benigne Natur der Knotenprobe mit mindestens 91% Spezifität und mindestens 83% Klassifizierungssensitivität für das normalisierte Expressionsniveau eines Satzes von 32 Genen bestimmt wird.

2. Verfahren nach Anspruch 1wobei, die Natur des Knotens und die Wahrscheinlichkeit der Malignität der getesteten Probe gemäß der Entscheidungsregel des Klassifikators bestimmt werden, wobei die Daten zum Expressionsniveau des Satzes aus 32 Genen und das Genexpressionsniveau jeder Probe des Knotens im Trainingssatz berücksichtigt werden, das für jede Probe des Trainingssatzes für denselben Satz aus 32 Genen und unter denselben Normalisierungsbedingungen bestimmt wurden.

3. Verfahren nach Anspruch 2, wobei die Natur und Malignitätswahrscheinlichkeiteiner Knotenprobe nach dem Maximum-Likelihood-Kriteriumfür ein probabilistisches Modell der normalisierten Expressionswerte eines Satzes von 32 Genen der Probe bestimmt werden.

4. Verfahren nach Anspruch 1, **dadurch gekennzeichnet, dass** RT-qPCR, Oligonukleotid-Mikroarrays oder RNA-Seq-Transkriptomsequenzierung zur Bestimmung des Expressionsniveaus eines Gensatzes verwendet werden

5. Verwendung des Satzes von Hybridisierungssonden Hs00611096_m1, Hs00163811_m1, Hs00218591_m1, Hs00989715_m1, Hs00229255_m1, Hs00174944_m1, Hs00244839_m1, Hs00152928_m1, Hs00171315_m1, Hs01034070_jn1, Hs00383314_m1, Hs01922255_s1, Hs00361131_g1, Hs00181853jn1, Hs00169943_m1, Hs00158127_m1, Hs01120412_m1, Hs01565584_m1, Hs00378551_m1, Hs01083647_m1, Hs01036720_m1, Hs01552593_m1, Hs00153649_m1, Hs00939867_m1, Hs01120908jfn1, Hs01070627_m1, Hs01104163_m1, Hs00377618_m1, Hs00268204_m1, Hs01547334_m1, Hs00892519_m1, Hs00185147_m1 zur Bestimmung des Expressionsniveaus eines entsprechenden Gens aus einem Satz von 32 Genen AMOT, C3, CAMK2N1, CFI, DCSTAMP, DIO1, DUSP5, EGR1, EMP2, FAM20A, FAXC, FLRT3, HBG1, IGFBP6, IGSF1, ITGA2, KCNQ3, MET, METTL7B, MPZL2, NOD1, OLR1, PDE5A, PSD3, SDC4, SLC26A4, SLC26A7, SLC5A8, SLPI, TM4SF1, TPO, TUSC3 und zuz Bestimmung der Natur des Knotens durch das Verfahren nach Ansprüchen 1 bis 3, unter Verwendung eines Computersoftwareprodukts mit einem computerlesbaren Medium und Ressourcen zum Messen des normalisierten Expressionsniveaus eines Gensatzes, zur Durchführung der Klassifikation und zur Bestimmung der Malignitätswahrscheinlichkeit einer Knotenprobe.

## Revendications

1. Une méthode de différenciation des nodules thyroïdiens bénins et malins dans les échantillons de biopsie tumorale, comprenant :
- étapes d'isolement de l'ARN, et
- détermination des niveaux d'expression des gènes et utilisation d'un classificateur
- comparison de l'expression génique de l'échantillon de nodules thyroïdiens avec l'expression génique des échantillons de nodules thyroïdiens dans le kit d'entraînement, puis
- identification de la nature maligne ou bénigne du nodule et determination de la probabilité de malignité du nodule thyroïdien,
avec le classificateur entraîné sur les données d'expression génique corrélées avec la nature du nodule thyroïdien, en particulier les nodules thyroïdiens bénins et les tumeurs malignes, y compris le carcinome thyroïdien papillaire et folliculaire,
**caractérisé en ce que**
le niveau d'expression d'un ensemble de 32 gènes est déterminé, qui se compose de AMOT, C3, CAMK2N1, CFI, DCSTAMP, DIO1, DUSP5, EGR1, EMP2, FAM20A, FAXC, FLRT3, HBG1, IGFBP6, IGSF1, ITGA2, KCNQ3, MET, METTL7B, MPZL2, NOD1, OLR1, PDE5A, PSD3, SDC4, SLC26A4, SLC26A7, SLCSA8, SLPI, TM4SF1, TPO et TUSC3,
et la normalisation de l'expression de chaque gène de cet ensemble est effectuée sous le contrôle endogène des six gènes de normalisation suivants EIF3A (EIF3S10), HADHA, UBE2D2, EIF5, PPIE et RPLPO,
et la nature maligne ou bénigne de l'échantillon de nodule est déterminée avec une spécificité d'au moins 91 % et une sensibilité de classification d'au moins 83 % pour le niveau d'expression normalisé d'un ensemble de 32 gènes,

2. Le procédé, selon la revendication 1, **caractérisé en ce que** la nature du nodule et la probabilité de malignité de l'échantillon testé sont déterminées selon la règle de décision du classificateur, en tenant compte des données de niveau d'expression de l'ensemble de 32 gènes et du niveau d'expression génique de chaque échantillon du nodule dans l'ensemble d'entraînement, déterminé pour chaque échantillon de l'ensemble d'entraînement pour le même ensemble de 32 gènes et dans les mêmes conditions de normalisation.

3. Le procédé selon la revendication. 2, **caractérisé en ce que** la nature et la probabilité de malignité d'un échantillon de nodule sont déterminées selon le critère de probabilité maximale pour un modèle probabiliste des valeurs d'expression normalisées d'un ensemble de 32 gènes de l'échantillon.

4. Le procédé selon la revendication. 1, **caractérisé en ce que** la RT-qPCR, les microréseaux d'oligonucléotides ou le séquençage du transcriptome ARN-seq sont utilisés pour détecter le niveau d'expression d'un ensemble de gènes.

5. Utilisation de l'ensemble de sondes d'hybridation Hs00611096_m1, Hs00163811_m1, Hs00218591_m1, Hs00989715_m1, Hs00229255_m1, Hs00174944_m1, Hs00244839_m1, Hs00152928_m1, Hs00171315_m1, Hs01034070_jn1, Hs00383314_m1, Hs01922255_s1, Hs00361131_g1, Hs00181853jn1, Hs00169943_m1, Hs00158127_m1, Hs01120412_m1, Hs01565584__m1, Hs00378551_m1, Hs01083647_m1, Hs01036720_m1, Hs01552593_m1, Hs00153649_m1, Hs00939867_m1, Hs01120908jfn1, Hs01070627_m1, Hs01104163_m1, Hs00377618_m1, Hs00268204_m1, Hs01547334_m1, Hs00892519_m1, Hs00185147_m1 pour détecter dans un échantillon de nodule thyroïdien le niveau d'expression d'un gène correspondant à partir d'un ensemble de 32 gènes AMOT, C3, CAMK2N1, CFI, DCSTAMP, DIO1, DUSP5, EGR1, EMP2, FAM20A, FAXC, FLRT3, HBG1, IGFBP6, IGSF1, ITGA2, KCNQ3, MET, METTL7B, MPZL2, NOD1, OLR1, PDE5A, DSP3, SDC4, SLC26A4, SLC26A7, SLC5A8, SLPI, TM4SF1, TPO, TUSC3 et pour déterminer la nature du nodule par le procédé selon les revendications 1 à 3, en utilisant un produit logiciel informatique ayant un support lisible par ordinateur et des ressources pour mesurer le niveau d'expression normalisé d'un ensemble de gènes, effectuer une classification et déterminer la probabilité de malignité d'un échantillon de nodule.
